# EUROPEAN PATENT APPLICATION

(11) **EP 2 077 105 A1**
(43) Date of publication of application: **08.07.2009**
(21) Application number: 08300003.4
(22) Date of filing: 02.01.2008
(51) Int. Cl.: A61K 9/107, A61K 47/18

(54) **Ophthalmic Micellar Compositions with Enhanced Stability**

(71) Applicant: Novagali Pharma S.A., 91000 Evry (FR)
(72) Inventor: Lambert, Grégory, 92290, CHATENAY MALABRY (FR); Lallemand, Frédéric, 94260, FRESNES (FR); Rabinovich-Guillatt, Laura, 60920, KADIMA (IL); Bague, Séverine, 91360, EPINAY SUR ORGE (FR); Garrigue, Jean-Sébastien, 91370, VERRIERE LE BUISSON (FR)
(74) Representative: Cabinet Plasseraud

(57) **Abstract**

This invention relates to micellar compositions comprising at least one pharmaceutically active substance and a mixture of n-alkyl dimethyl benzyl ammonium chlorides, wherein the mixture comprises more than 30% n-alkyl dimethyl benzyl ammonium chlorides having a chain length superior or equal to C16, wherein the pharmaceutically active substance exhibits an improved stability. In particular, a composition is provided that comprises cetalkonium chloride micelles incorporating the prostaglanding-like therapeutic agent, latanoprost. This invention also relates to ophthalmic compositions containing such micellar compositions and methods of using these ophthalmic compositions for the treatment of eye conditions.

## Description

The present invention relates to cationic micellar compositions, in particular to micellar compositions of n-alkyl dimethyl benzyl ammonium chlorides, comprising at least one pharmaceutically active substance. These micellar compositions are useful for eye care and for the treatment of eye conditions.

Quaternary ammonium compounds are organic molecules generally used as antiseptic or antimicrobial agents. Benzalkonium chloride, a nitrogenous cationic surface-active agent belonging to the family of quaternary ammonium compounds, is the most commonly used preservative in ophthalmology. About 70% of all eye drop specialities currently available contain benzalkonium chloride as preservative, at a concentration generally comprised between 0.01% and 0.02% weight/volume.

Benzalkonium chloride, as usually provided by manufacturers wanting to comply with the European and/or American Pharmacopeia, is a mixture of alkylbenzyl dimethylammonium chlorides of general formula: C₆H₅CH₂N(CH₃)2RCl, wherein R is a C₁₂-C₂₄ alkyl group. Generally, the main components of the mixture have chain lengths of C₁₂, C₁₄ and C₁₆. European and US Pharmacopeia require that C₁₂-alkyl dimethyl benzyl ammonium chlorides represent at least 40% of the mixture, C₁₄-alkyl dimethyl benzyl ammonium chlorides represent at least 20%, with the sum of the C₁₂ and C₁₄ species representing at least 70% of the mixture.

The biocidal activity of benzalkonium chloride is thought to be due to its ability to disrupt cell membranes of pathogens, which compromises cellular permeability control and induces leakage of cellular contents. This ability to disrupt cell membranes is also thought to be responsible, at least in part, for the enhancement in corneal permeability observed after instillation of benzalkonium chloride. Indeed, ophthalmic formulations preserved by benzalkonium chloride generally exhibit an improved corneal and conjunctival penetration with consequent improved pharmaceutical activity. Thus, in such compositions, in addition to acting as a preservative, benzalkonium chloride also plays a role of penetration enhancer (K. Okabe et al., Invest. Ophthalmol. Vis. Sci., 2005, 46: 703-708).

Benzalkonium chloride micelles have been used for the formulation of ophthalmic compositions of prostaglandins. Prostaglandins are known to efficiently lower the intraocular pressure (IOP) in several species including primates. In particular, benzalkonium chloride micelles have been used in the preparation of ophthalmic compositions of Latanoprost, a prostaglandin-like therapeutic agent useful in the treatment of glaucoma. Latanoprost is a selective FP receptor agonist, which lowers intraocular pressure by promoting outflow of an aqueous humor.

An ophthalmic composition comprising Latanoprost (0.005% w/v) in benzalkonium chloride micelles (0.02% w/v) is commercially available under the trade name Xalatan (U.S. Pat. Nos. 4,599,353; 5,296,504; and 5,422,368). The pharmacological activity of Xalatan may result from the penetration enhancing activity of benzalkonium chloride by disruption of epithelial cell membranes at the surface of the cornea, and/or from the high affinity of benzalkonium chloride cationic micelles for the negatively charged corneal surface, allowing an exchange of latanoprost from the micelles to the cornea.

Xalatan is indicated for the reduction of elevated intraocular pressure in patients with open-angle glaucoma or ocular hypertension. However, this commercially available ophthalmic solution suffers from several limitations. In particular, it lacks stability, requiring storage in a cold environment (2°C to 8°C) shielded from exposition to light. Furthermore, it is also significantly toxic to the ocular surface, causing superficial irritation and vasodilation in the conjunctiva.

Therefore, there is still a need in the art for improved formulation approaches to overcome the above-mentioned problems. Particularly desirable is the development of formulations whose biodisponibility does not depend on a mechanism that is associated with high toxicity.

Thus, one of the goals of the present invention is to provide micellar compositions comprising a reduced amount of quaternary ammonium compounds and that, therefore, exhibit lower toxicity than compositions comprising benzalkonium chloride. Such micellar compositions have the advantage that they may be unpreserved. In addition, they better prevent degradation of any solubilised active principle than micelles of benzalkonium chloride.

The present Applicants have observed that, in emulsions, quaternary ammonium compounds with long alkyl chains (for example quaternary ammonium compounds having C₁₄-C₁₈ alkyl chains) when compared to C₁₂-alkyl chains, do not exhibit good bactericidal properties but confer a greater cationic power.

Thus, the present invention encompasses the recognition by the Applicants that mixtures of n-alkyl dimethyl benzyl ammonium chlorides comprising more than 30% (i.e., > 30%) of n-alkyl dimethyl benzyl ammonium chlorides having a chain length superior or equal to C₁₆ can be advantageously used in place of benzalkonium chloride.

The term "n-alkyl dimethyl benzyl ammonium chloride having a chain length superior or equal to C₁₆" refers to an n-alkyl dimethyl benzyl ammonium chloride wherein the alkyl chain comprises 16 carbon atoms, or more than 16 carbon atoms, *e*.*g*., 17, 18, 19, 20, 21 or more than 21 carbon atoms.

For example, when the alkyl chain comprises 16 carbon atoms, the corresponding n-alkyl dimethyl benzyl ammonium chloride is called cetalkonium chloride (CKC). CKC is the C₁₆ component of benzalkonium chloride. The terms "cetalkonium chloride", "CKC", and "BAK C16" are used herein interchangeably and refer to compound CAS 122-18-9. Like benzalkonium chloride, CKC may form cationic micelles; however, it is more lipophilic than benzalkonium chloride. This increase in lipophilicity allows the use of lower concentrations of cationic surfactant without a decrease in the cationic charge on the surface of micelles. Thus, toxicity is minimized while biodisponibility of any entrapped drug is not affected.

In one aspect, the present invention provides a micellar composition comprising a mixture of n-alkyl dimethyl benzyl ammonium chlorides comprising more than 30% of n-alkyl dimethyl benzyl ammonium chlorides having a chain length superior or equal to C₁₆, wherein at least some of the n-alkyl dimethyl benzyl ammonium chlorides are present at a concentration above their critical micelle concentration to form micelles. Preferably, micellar compositions provided herein are useful for ophthalmic or cosmetic purposes.

The term "micelle" has its art understood meaning and refers to an aggregate of surfactant molecules dispersed in a liquid colloid. A typical micelle in aqueous solution forms an aggregation with the hydrophilic "head" regions in contact with the surrounding solvent, sequestering the hydrophobic tail regions in the micelle center. This type of micelle is known as a normal phase micelle (or oil-in-water micelle). Inverse micelles have the head groups at the center with the tails extending out (water-in-oil micelle). In the present invention, micelles are generally normal phase micelles.

The shape and size of a micelle is a function of the molecular geometry of the surfactant molecules and solution conditions such as surfactant concentration, temperature, pH, and ionic strength. Micelles are generally spherical in shape, but other shapes such as ellipsoids, cylinders, and bilayers are also possible. Micelles of the present invention will preferably be substantially spherical in shape. Preferably, when a composition of the present invention comprises a plurality of populations of micelles, micelles of the main population (*i*.*e*., micelles of the population with the largest number of micelles) have a mean diameter of between about 1 nm and about 100 nm, preferably between about 1 nm and about 50 nm, more preferably between about 1 nm and about 20 nm.

The terms "approximately" and "about", as used herein in reference to a number, generally includes numbers that fall within a range of 10% in either direction of the number (greater than or less than the number) unless otherwise stated or otherwise evident from the context (except where such a number would exceed 100% of a possible value).

As used herein, the term "critical micelle concentration" has its art understood meaning and refers to the concentration of a surfactant above which micelles are spontaneously formed. Cetalkonium chloride, for example, has a critical micelle concentration of about 0.0090% w/w.

More specifically, the present invention provides a composition comprising a mixture of n-alkyl dimethyl benzyl ammonium chlorides, as described above, wherein the mixture is present at a concentration below 0.02% w/w. Unless otherwise stated, percentages are herein expressed in weight relative to the total weight of the composition (% w/w). More specifically, compositions of the present invention generally comprise the mixture of n-alkyl dimethyl benzyl ammonium chlorides at a concentration comprised between about 0.0090% and about 0.02% w/w. For example, the mixture may be present at a concentration between about 0.004% and about 0.015% w/w, or between about 0.005% and about 0.01% w/w, *e*.*g*., 0.005%, 0.006%, 0.007%, 0.008%, 0.009% or 0.01% w/w.

Micelles of the present invention are cationic micelles, *i*.*e*., micelles that are made up of amphiphilic molecules with polar groups that are capable of being positively charged at or around physiological pH. This property is understood in the art to be important in defining how the amphiphilic molecules (and consequently the cationic micelles) interact with other molecules, including biomolecules.

Like cationic emulsions, cationic micelles are particularly useful as topical ophthalmic vehicles since they have the advantage of increasing the bioavailability of entrapped drugs by electrostatic attraction between the vehicle's positive charge and the negative charges carried by the eye surface. Furthermore, the presence of positive electrostatic charges at the surface of micelles causes micelle repulsions and reduces micelle coalescence, resulting in stabilization of the micellar composition.

Thus in preferred embodiments, micellar compositions of the present invention have a positive zeta potential. As known in the art, the zeta potential is a measure of the magnitude of repulsion or attraction between particles (Washington, Adv. Drug Deliv. Reviews, 1996, 20:131-145). The Zeta potential is not measurable directly but it can be calculated using theoretical models and an experimentally-determined electrophoretic mobility or dynamic electrophoretic mobility. As known in the art, electrophoretic mobility can be determined using micro-electrophoresis or electrophoretic light scattering.

Mixtures of n-alkyl dimethyl benzyl ammonium chlorides used in compositions of the present invention generally comprise more than 30% of n-alkyl dimethyl benzyl ammonium chlorides having a chain length superior or equal to C₁₆, *i*.*e*., 31% or more, *e*.*g*., between about 35% and about 50%, or between about 40% and about 60%, or between about 50% and about 70%, or between about 60% and about 80%, or between about 70% and about 90%, or more than about 90%.

Micellar compositions of the present invention contain at least one pharmaceutically active substance associated with micelles of n-alkyl dimethyl benzyl ammonium chlorides.

The term "associated with micelles of n-alkyl dimethyl benzyl ammonium chlorides", as used herein in connection with a pharmaceutically active substance, refers to a substance that is linked, bound or otherwise attached at the surface of the micelles, and/or that is embedded, entrapped or incorporated into the micelle core.

As known in the art, when surfactants such as CKC are present above their critical micelle concentration, they act as emulsifiers that will allow a compound normally insoluble (in the solvent being used) to become solubilized. This occurs because the insoluble species can be incorporated into the micelle core, which is itself solubilized in the bulk solvent by virtue of the head groups' favorable interactions with solvent molecules.

In certain preferred embodiments, a pharmaceutically active substance is, substantially, embedded, entrapped or incorporated into the micelle core (as opposed to linked, bound or attached to the micelle surface). For example, more than about 50% of the substance present in the composition is incorporated into the micelle core, preferably more than about 75% of the substance is incorporated into the micelle core, most preferably, more than about 80% of the substance is incorporated into the micelle core *e*.*g*., more than about 85%, 90%, 95%, 99% or more than 99%.

Pharmaceutically active substances suitable for use in the present invention may be found among a wide variety of molecules, compounds, agents, or factors effective in the management or treatment of a disease or clinical condition. In certain preferred embodiments, such pharmaceutically active substances are poorly water-soluble.

For example, active substances may be selected from different families of drugs including, but not limited to, antibiotics (*e*.*g*., aminoglycosides, carbacephem, carbapenems, cephalosporins, glycopeptides, penicillins, polypeptides, quinolones, sulfonamides, tetracyclines and the like); antiviral agents (*e*.*g*., cidofovir, ganciclovir, valaciclovir or acyclovir); antifungals (*e*.*g*., polyene antibiotics, imidazole and triazole, allylamines); intraocular pressure lowering agents (*e*.*g*., alpha-adrenergic agonists, beta-adrenergic blockers, carbonic anhydrase inhibitors, cannabinoids, derivatives and prodrugs); anti-inflammatory agents including non-steroidal anti-inflammatory agents (*e*.*g*., COX-2 inhibitors, salicylates, 2-arylpropionic acids, N-arylanthranilic acids, oxicams, sulphonanilides, pyrazolidines derivatives, arylalkanoic acids, 3-benzolphenylacetic acids and derivatives); steroids (*e*.*g*., cortisone, hydrocortisone, prednisone, prednisolone, methylprednisone, fluoromethalone, medrysone, betamethasone, loteprednol, flumethasone, mometasone, testosterone, methyltestosterone, danazol, beclomethasone, dexamethasone, dexamethasone palmitate, tramcinolone, triamcinolone acetonide, fluocinolone, fluocinolone acetonide, difluprednate); antiallergic compounds (*e*.*g*., olapatadine, ketotifen, azelastine, epinastine, emedastine, levocabastive, terfenadine, astemizole and loratadine); anti-angiogenic compounds (*e*.*g*., thalidomide, VEGF inhibitors, VEGF soluble receptors, VEGF-traps, VEGF-antibodies, VEGF-traps, anti VEGF-siRNA); biological agents (*e*.*g*., antibodies or antibodies fragments, oligoaptamers, aptamers and gene fragments, oligonucleotides, plasmids, ribozymes, small interference RNA, nucleic acid fragments, peptides and antisense sequences); growth factors (*e*.*g*., epidermal growth factor, fibroblast growth factor, platelet derived growth factor, transforming growth factor beta, ciliary neurotrophic growth factor, glial derived neurotrophic factor, NGF, EPO and P1GF); immunomodulating agents (*e*.*g*., glucocorticoids, drugs acting on immunophilins, interferons, opioids); cytostatics (*e*.*g*., alkylating agents, antimetabolites and cytotoxic antibiotics); antioxidants (*e*.*g*., alpha-tocopherol, ascorbic acid, retinoic acid, lutein and their derivatives, precursors or prodrugs); UV-filter compounds (*e*.*g*., benzophenones); anti-redness agents (*e*.*g*., naphazoline, tetrahydrozoline, ephedrine and phenylephrine); fatty acids (*e*.*g*., omega-3 fatty acids), and the like, and any combinations thereof.

In certain embodiments, the pharmaceutically active substance associated with micelles is a non-steroidal anti-inflammatory agent, *e*.*g*., flubiprofen.

In certain embodiments, pharmaceutically active substances associated with micelles are anti-glaucomateaous active substances that can be selected among beta-blockers (*e*.*g*., levobunolol, befundol, metipranolol, forskolin, cartrolol, timolol); inhibitors of carbonic anhydrase (*e*.*g*., brinzolamide, dorzolamide, acetazolamide, methazolamide, dichloro-phenamide); sympathomimetics (*e*.*g*., brimonidine, apraclonidine, dipivefrine, epinephrine); parasympathomimetics (*e*.*g*., pilocarpine); or cholinesterase inhibitors (*e*.*g*., physostigmine, echothiophate and/or their derivatives and/or pharmaceutically acceptable salts thereof).

The amount of pharmaceutically active substance(s) present in a micellar composition of the present invention will depend on the nature of the active substance, as well as the size of micelles. In general, the amount of pharmaceutically active substance(s) may be between of about 0.001% and about 1% w/w.

In certain preferred embodiments, micellar compositions of the present invention comprise at least one prostaglandin, as pharmaceutically active substance. Preferably, the prostaglandin is associated with micelles of n-alkyl dimethyl benzyl ammonium chlorides. More preferably, the prostaglandin is, substantially, incorporated into the core of micelles of n-alkyl dimethyl benzyl ammonium chlorides. The term "prostaglandin", as used herein, refers indifferently to prostaglandin, precursors, derivatives or analogs thereof.

The use of prostaglandin analogs for the treatment of glaucoma and ocular hypertension is known in the art (see, for example, U.S. Pat. Nos. 4,599,353; 5,849,792; 5,688,819 and 6,011,062).

Prostaglandins suitable for use in the practice of the present invention may be prostaglandin D₂ analogs, prostaglandin E₂ analogs, prostaglandin F_{2α} analogs, or any combination thereof. The present invention is of particular interest for prostaglandin F_{2α} analogs. Thus, in certain embodiments, compositions of the present invention comprise at least one prostaglandin F_{2α} analog such as, for example, latanoprost, unoprostone isopropyl, travoprost, bimatoprost, tafluprost, 8-isoprostaglandin E2, or any combination thereof.

In certain embodiments, latanoprost is the only pharmaceutically active substance in a micellar composition of the present invention. In other embodiments, latanoprost is present in combination with at least one additional prostaglandin, for example a prostaglandin E₂ analog such as unoprostone isopropyl, travoprost, bimatoprost, tafluprost, or 8-isoprostaglandin E2. Alternatively or additionally, latanoprost may be present in combination with at least one additional pharmaceutically active substance such as those described herein.

The amount of prostaglandins present in a micellar composition of the present invention will depend on the nature of the prostaglandin(s) and the intended use of the micellar composition, as well as on the size of micelles. In certain embodiments, the amount of prostaglandin or mixture thereof is comprised between about 0.001% and about 1% w/w, preferably between about 0.002% and about 0.1% w/w, and even more preferably between about 0.002% and about 0.01% w/w.

In certain embodiments, a pharmaceutically active substance comprised in a micellar composition of the present invention is "stabilized". As used in the context of the present invention, the term "stabilized" means that in an inventive micellar composition, the pharmaceutically active substance exhibits an "enhanced stability", "improved stability" or "increased stability" compared to its stability in currently available formulations for ophthalmic topical administration, in particular formulations that comprise BAK. Without being bound by any theory, it is believed that since the pharmaceutically active substance is solubilised in the micelle core, it is less available to contact with agents enhancing its degradation. "Stability" is defined as the extent to which a product retains, within specified limits and throughout its period of storage and use (*i*.*e*., its shelf life), the same properties and characteristics that it possessed at the time of manufacture. One of the purposes of stability testing is to provide evidence on how the quality of a drug substance or drug product varies overtime under the influence of a variety of environmental factors such as temperature, humidity and light. The results of such testing enable recommended storage conditions, re-test periods, and shelf lives to be established.

Although real-time stability studies include an evaluation of those factors that ultimately affect the expiration date of drugs, they are time- and cost-consuming. Conventionally, accelerated stability studies are used for predicting the shelf life of pharmaceutical products. Such accelerated studies generally submit the systems tested to a temperature of 40°C for 6 or 9 months.

In certain embodiments, a micellar composition of a pharmaceutically active substance according to the present invention is stable for more than about 1 year, preferably more than about 2 years, most preferably more than about 3 years, when stored at room temperature.

Pharmaceutically active substances that are known to be unstable under storage conditions may be found in a wide variety of families of drugs, including those described above. In certain embodiments, such unstable pharmaceutically active substances are selected among prostaglandins, for example, prostaglandin F_{2α} analogs, *e*.*g*., latanoprost, unoprostone isopropyl, travoprost, bimatoprost, tafluprost, 8-isoprostaglandin E2, derivatives thereof, or any combinations thereof. In certain preferred embodiments, the pharmaceutically active substance is latanoprost, which has been shown to be unstable, for example, when formulated in benzalkonium chloride micelles.

According to another aspect, the present invention relates to pharmaceutical compositions comprising an effective amount of a micellar composition disclosed herein and at least one pharmaceutically acceptable carrier or excipient.

As used herein, the term "effective amount" refers to any amount of a compound, agent or composition that is sufficient to fulfill its intended purpose(s), *e*.*g*., a desired biological or medicinal response in a tissue, system or subject. For example, in certain embodiments of the present invention, the purpose(s) may be: to slow down or stop the progression, aggravation, or deterioration of the symptoms of a disease (*e*.*g*., inflammation, allergy, dry eye, retinal diseases, infections, glaucoma, or ocular hypertension), to bring about amelioration of the symptoms of the disease, and/or to cure the disease.

The term "pharmaceutically acceptable carrier or excipient" refers to an agent or medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, adsorption delaying agents, and the like. The use of such media and agents for pharmaceutically active substances is well known in the art (see for example, "Remington's Pharmaceutical Sciences", E.W. Martin, 18th Ed., 1990, Mack Publishing Co.: Easton, PA, which is incorporated herein by reference in its entirety). In particular, in certain preferred embodiments, a pharmaceutically acceptable carrier or excipient does not substantially affect the zeta potential of micelles of n-alkyl dimethyl bezyl ammonium chlorides overtime, or the amount of pharmaceutically acceptable carriers or excipients that can affect the zeta potential of micelles is such that at any time, the amount of positive charges is above the amount of negative charges. Substances susceptible of affecting the zeta potential of cationic micelles may be any substance that is negatively charged or that becomes negatively charged overtime.

In certain embodiments, the present invention provides a pharmaceutical composition comprising:
a) a micellar composition comprising a mixture of n-alkyl dimethyl benzyl ammonium chlorides at a concentration of less than 0.02% in weight by weight of the total composition and at least one pharmaceutically active substance, wherein the mixture of n-alkyl dimethyl benzyl ammonium chlorides comprises more than 30% of n-alkyl dimethyl benzyl ammonium chlorides having a chain length superior or equal to C₁₆,
b) optionally, one or more additional surfactants,
c) optionally, one or more of: antioxidants, isotonicity, thickening/viscosifying, preservative, pH adjusting, buffering, solubilising, chelating, and penetration enhancing agents, and
d) water.

In certain embodiments, pharmaceutical compositions of the present invention comprise one or more additional surfactants. Surfactants that are suitable for use in the preparation of such compositions include any non-ionic or cationic surfactants, which when combined to the mixture of n-alkyl dimethyl benzyl ammonium chlorides, lead to the formation of cationic micelles that present a high affinity for the corneal surface. Examples of non-ionic surfactants that can be present in a pharmaceutical composition of the present invention include, but are not limited to, poloxamers, tyloxapol, polysorbates (*e*.*g*., polysorbate 80), polyoxyethylene castor oil derivatives, derivatives of cremophors (*e*.*g*., cremophor EL, and cremophor RH), sorbitan esters, polyoxyl stearates, cremophors (*e*.*g*., cremophor EL, and cremophor RH), and combinations thereof. Examples of cationic agents that are suitable for use in the present invention include, but are not limited to, C₁₀-C₂₄ primary alkylamines, tertiary aliphatic amines, quaternary ammonium compounds selected from the group comprising lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristalkonium halide, stearalkonium halide or a mixture of two or more thereof, halide being preferably chloride or bromide, cationic lipids, amino alcohols, biguanide salts selected from chlorhexidine and salts thereof, polyaminopropyl biguanide, phenformin, alkylbiguanide or a mixture of two or more thereof, cationic compounds selected from 1,2-dioleyl-3-trimethyl-ammoniumpropane, 1,2-dioleoyl-sn-glycerol-phosphatidylethanolamine, cationic glycosphingolipids or cationic cholesterol derivatives, and any combinations thereof. Additional surfactants may be present at a concentration between about 0.01% and about 1% w/w.

Antioxidants suitable for use in the practice of the present invention include, but are not limited to, alpha-tocopherol and sodium bisulfate. Antioxidants may be present at a concentration between about 0.0001% and about 0.1% w/w.

Examples of suitable isotonic agents include, but are not limited to, mannitol, glycerol, sodium chloride, and dextrose. Isotonic agents may be present at a concentration between about 0.9% and about 5% w/w.

Pharmaceutical compositions of the present invention may comprise one or more thickening agents, for example viscosifying agents. Examples of viscosifying agents include, but are not limited to, sugars, such as sucrose, glucose, maltose, dextrose and fructose; hydric alcohols, such as sorbitol, mannitol, xylitol and maltitol; and polymers such as polydextrose, xanthan gum, guar gum, sodium alginate, carrageenan, hydroxypropyl, cellulose (HPC), hydroxypropyl cellulose (HPC), hydroxypropyl methylcellulose (HPMC), methylcellulose, polyvinylpyrrolidone (PVP), maltodextrin, carbomer, polyvinyl alcohol, polyethylene glycol (PEG), polyethylene oxide, carboxymethylcellulose (CMC), hydroxyethyl cellulose (HEC), and any combination thereof. Viscosifying agents may be present at a concentration between about 0.1% and about 6.0% w/w.

Examples of pH adjusting agents include, but are not limited to, acetates, citrates, phosphates, hydrochloride acid, and sodium hydroxide. pH adjusting agents are generally present at a concentration sufficient to adjust the pH of a composition to a desired value.

Examples of preservatives suitable for use in the present invention include, but are not limited to, sodium benzoate, methylparaben, propylparaben, polyhexamethylene, biguanide, sodium perborate, and the like. Preservatives may be present at a concentration between about 0.001% and about 0.5% w/w.

Examples of chelating agents include, but are not limited to, ethylenediaminetetraacetic acid and edetate disodium. Chelating agents may be present at a concentration between about 0.01% and about 0.5% w/w.

Examples of penetration enhancers include, but are not limited to, certain organic solvents, such as dimethylsulfoxide and other sulfoxides, dimethylacetamide and pyrrolidone; certain amides of heterocyclic amine, glycols (*e*.*g*., propylene glycol); propylene carbonate; oleic acid; alkyl amines and derivatives. Penetration enhancers may be present at a concentration between about 0.1% and about 3% w/w.

A pharmaceutical composition according to the present invention may further comprise one, or more than one, additional therapeutic agent that is not associated with micelles of n-alkyl dimethyl benzyl ammonium chlorides. Suitable therapeutic agents include any of a wide variety of pharmaceutically active substances (such as those described herein) whose therapeutic activity is beneficial to the subject to whom the composition is administered.

Micellar compositions of the present invention may be obtained by mixing the various components of the composition. The different components may be added to the reaction mixture in any suitable order (*e*.*g*., one after the other or all together at the same time). In certain embodiments, quaternary ammonium compounds and a pharmaceutically active substance are preferably added to the reaction mixture at the same time, to allow for incorporation of the substance into the micelles' core.

As already mentioned above, micelles only form when the concentration of surfactant is greater than the critical micelle concentration, and the temperature of the system is greater than the critical micelle temperature, or Krafft temperature. Optimization of reaction conditions for the preparation of micellar compositions according to the present invention is within the knowledge of one skilled in the art.

Components of compositions of the present invention may be synthesized using methods and procedures known in the art and/or may be purchased from commercial sources and optionally purified before use.

In certain embodiments, after preparation and before use, a composition of the present invention is sterilized. Sterilization may be carried out using any suitable method, for example, by filter sterilization.

Alternatively or additionally, micellar compositions described herein may be freeze-dried or lyophilized for long-term storage, if desired. In such embodiments, it may be desirable to include a cryoprotectant for stabilization during lyophilisation. Alternatively, the physical structure of the micelles can be preserved by the presence of sufficient water after lyophilisation. This may be accomplished by appropriate control of the degree of lyophilisation. Any cryoprotective agent known to be useful in the art of preparing freeze-dried formulations, such as di- or polysaccharides or other bulking agents such as lysine, may be used in the claimed invention. If an inventive composition is lyophilized, it may be packed in vials for subsequent reconstitution with an aqueous solution, such as sterile water or sterile water containing a saccharide and/or other suitable excipients, just prior to use. For example, reconstitution may be by simply adding water before topical application.

Micellar compositions of the present invention are useful for eye care and for the treatment of eye diseases or eye conditions.

As used herein, the term "eye disease or eye condition" refers to any of a wide variety of ocular conditions such as glaucoma, ocular inflammatory conditions such as keratitis, uveitis, intra-ocular inflammation, allergy and dry-eye syndrome ocular infections, ocular allergies, ocular infections, cancerous growth, neo vessel growth originating from the cornea, retinal oedema, macular oedema, diabetic retinopathy, retinopathy of prematurity, degenerative diseases of the retina (macular degeneration, retinal dystrophies), and retinal diseases associated with glial proliferation.

Thus, in certain preferred embodiments, compositions of the present invention are formulated for topical administration to the eye and are in the form of eye drops, eye ointment, or ophthalmic gels.

The term "ophthalmic", as used herein in connection with a composition, refers to a composition intended to be administered to the eye and which presents a pharmaceutical effect.

Pharmaceutical compositions of the present invention may be formulated in dosage unit form for ease of administration and uniformity of dosage. The expression "unit dosage form", as used herein, refers to a physically discrete amount of a micellar composition to treat a patient. It will be understood, however, that the total daily usage of compositions of the present invention will be decided by the attending physician within the scope of sound medical judgement.

Thus, according to this aspect, the present invention relates to the use of micelles described herein for the preparation of a pharmaceutical composition or medicament for the treatment of an eye disease or condition. In certain preferred embodiments, the eye disease or condition is a member of the group consisting of inflammation, allergy, dry eye, retinal diseases, infections, glaucoma, and ocular hypertension.

In a related aspect, the present invention relates to methods of treatment of eye diseases or conditions, said methods comprising a step of administering to a subject in need thereof a micellar composition described herein. Administration is generally performed by topical application to the eye of the subject.

The terms "subject" and "individual" are used herein interchangeably. They refer to a human or another mammal (*e*.*g*., mouse, rat, rabbit, dog, cat, cattle, swine, sheep, horse, or primate) that can be afflicted with or is susceptible to an eye disease or condition but may or may not have the disease or condition. In many embodiments, the subject is a human being. The terms "individual" and "subject" do not denote a particular age, and thus encompass adults, children, and newborns.

A treatment according to the present invention may consist of a single dose or a plurality of doses over a period of time. Administration may be one or multiple times daily, weekly (or at some other multiple day interval) or on an intermittent schedule.

Optimization of the appropriate dosages can readily be made by one skilled in the art in light of pharmacokinetic data observed in clinical trials. Final dosage regimen will be determined by the attending physician, considering various factors which modify the action of the drug, *e*.*g*., the drug's specific activity, the severity of the disease or condition and the responsiveness of the patient, the age, condition, body weight, sex and diet of the patient, the severity of any present infection, time of administration, the use (or not) of concomitant therapies, and other clinical factors. As studies are conducted using compositions of the present invention, further information will emerge regarding the appropriate dosage levels and duration of treatment.

It will be appreciated that pharmaceutical compositions of the present invention can be employed alone or in combination with additional therapies. In other words, a treatment according to the present invention can be administered concurrently with, prior to, and/or subsequently to one or more desired therapeutics or medical procedures. The particular therapies (therapeutic or procedures) to employ in such a combination regimen will take into account compatibility of the desired therapeutics and/or procedures and the desired therapeutic effect to be achieved.

For example, an inventive method for the treatment of glaucoma may be used in combination with topical eye medications (*e*.*g*., levobunolol (Betagan), timolol (Betimol, Timoptic), carteolol (Ocupress), betaxolol (Betoptic), metipranolol (OptiPranolol), apraclonidine (Iopidine), brimonidine (Alphagan), dorzolamide (Trusopt), brinzolamide (Azopt), and/or pilocarpine (Isopto Carpine, Pilocar)); systemic medications (*e*.*g*., acetazolamide and/or methazolamide); laser treatment (*e*.*g*., trabeculoplasty); surgery (*e*.*g*., trabeculectomy and/or drainage implant surgery), or any combination of these treatments.

In still another aspect, the present invention relates to pharmaceutical packs or kits. A pharmaceutical pack or kit according to the present invention comprises one or more containers (*e*.*g*., vials, ampoules, test tubes, flasks or bottles) containing one or more ingredients of an inventive composition, allowing administration to a subject. Such containers may be made of glass, plastic materials, resins, and the like. They may be transparent or, alternatively, they may be colored or opaque to prevent or reduce direct exposition to light. In certain embodiments, a container is in a form that allows for administration of a controlled volume (*e*.*g*., a drop) of micellar composition. In other embodiments, a container comprises a system (*e*.*g*., a dropper) allowing administration of a controlled volume of micellar composition.

Different ingredients of a pharmaceutical pack or kit may be supplied in a liquid form or in a solid form (*e*.*g*., lyophilized). Each ingredient will generally be suitable as aliquoted in its respective container or provided in a concentrated form. Pharmaceutical packs or kits may include media for the reconstitution of lyophilized ingredients. Individual containers of a kit will preferably be maintained in close confinement for commercial sale.

In certain embodiments, a pharmaceutical pack or kit includes one or more additional approved pharmaceutically active substances, such as those described above. Optionally associated with such container(s) can be a notice or package insert in the form prescribed by a governmental agency regulating the manufacture, use or sale of pharmaceutical or biological products, which notice reflects approval by the agency of manufacture, use or sale for human administration. The notice or package insert may contain instructions for use of a pharmaceutical composition according to methods disclosed herein.

An identifier, *e*.*g*., a bar code, radio frequency, ID tags, etc., may be present in or on the kit. The identifier can be used, for example, to uniquely identify the kit for purposes of quality control, inventory control, tracking movement between workstations, etc.

The following examples and figures illustrate the invention and its improved ocular tolerance compared to currently available products. However, these examples and figures should not be interpreted in any way as reducing the scope of this invention.

Fig.1 is a set of pictures demonstrating the effects of 3 different eye drops on an acute toxicity rabbit model (see Example 5 for details). The first row shows pictures of rabbit eyes treated by PBS, Xalatan, and an inventive micellar composition of Latanoprost (Micelle #4). The second and third rows present microphotographs showing epithelial cells of these eyes in the surface epithelium and the basal epithelium, respectively. The photographs were taken 4 hours after treatment.

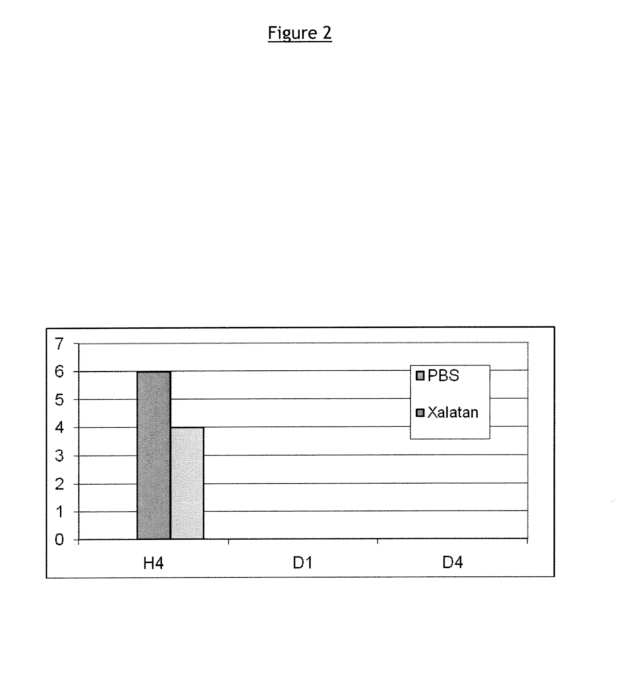

Fig. 2 is a graph showing the results of a Draize test criteria scoring carried out 4 hours (H4), 1 day (D1) or 4 days (D4) after rabbits had been treated with PBS, Xalatan, or an inventive micellar composition of Latanoprost (see Example 5 for details). For PBS, the score obtained was zero.

Fig. 3 is a graph showing the results of a HRT scoring test carried out 4 hours (H4), 1 day (D1) or 4 days (D4) after rabbits had been treated with PBS, Xalatan, or an inventive micellar composition of Latanoprost (see Example 5 for details).

Fig. 4 is a graph showing the percentage of TNFR1 positive cells in the rabbits conjunctiva determined 4 hours (H4) or 1 day (D1) after rabbits had been treated with PBS, Xalatan, or an inventive micellar composition of Latanoprost (see Example 5 for details).

Fig. 5 is a graph showing the percentage of RLA-DR positive cells in the rabbits conjunctiva determined 4 hours (H4) or 1 day (D1) after rabbits had been treated with PBS, Xalatan, or an inventive micellar composition of Latanoprost (see Example 5 for details).

### Examples

Unless otherwise stated, all concentrations in the compositions described below are expressed in weight/weight of the entire formulation percentages.

### Example 1: Micellar Composition Comprising Cetalkonium Chloride (CKC) Alone

A micellar composition (Micelle #1) was prepared that comprises 0.005% latanoprost, 0.01% CKC, and 0.9% NaCl in water. Results of a stability analysis carried out for this composition are presented in Table 1.

**Table 1. Stability of Composition Micelle #1**

| **Parameters** | T0 | T1month 25°C | T1month 40°C | T9months 25°C | T9months 40°C |
|---|---|---|---|---|---|
| **Size (nm)** | 17 (81%) 484 (13%) 1764 (6%) | 22 (79%) 2427 (20%) | 19 (82%) 430 (17%) | 18 (87%) 446 (12%) | 18 (95%) 1642 (4%) |
| **pH** | 6.15 | 6.45 | 6.06 | 5.97 | 5.95 |
| **Osmolality** | 290 | 288 | 289 | 287 | 285 |
| **Latanoprost assay % w/w (% from T0)** | 0.00500 | 0.00473 | 0.00486 | 0.00484 | 0.00470 |
| | | (94.5%) | (102.9%) | (102.4%) | (99.5%) |

### Example 2: Micellar Composition Comprising Cetalkonium Chloride (CKC) and Cremophor RH

A micellar composition (Micelle #2) was prepared that comprises 0.005% latanoprost, 0.1% Cremophor RH, 0.01% CKC, and 0.9% NaCl in water. Results of a stability analysis carried out for this composition are presented in Table 2.

**Table 2. Stability of Composition Micelle #2**

| **Parameters** | T0 | T1month 25°C | T1month 40°C | T9months 25°C | T9months 40°C |
|---|---|---|---|---|---|
| **Size (nm)** | 14 (96%) | 14 (91%) | 14 (95%) | 13 (100%) | 13 (100%) |
| | 1941 (3%) | 1923 (8%) | 2007 (4%) | | |
| **pH** | 6.05 | 6.19 | 6.12 | 5.79 | 5.77 |
| **Osmotatity** | 295 | 293 | 295 | 289 | 291 |
| **Latanoprost assay % w/w (% from T0)** | 0.00503 | 0.00552 | 0.00548 | 0.00557 | 0.00559 |
| | | (109.6%) | (99.5%) | (100.9%) | (101.3%) |

### Example 3: Micellar Composition Comprising Cetalkonium Chloride (CKC) at 0.01% and Tyloxapol

A micellar composition (Micelle #3) was prepared that comprises 0.005% latanoprost, 0.1% Tyloxapol, 0.01% CKC, and 0.9% NaCl in water. Results of a stability analysis carried out for this composition are presented in Table 3.

**Table 3. Stability of Composition Micelle #3**

| **Parameters** | T0 | T1month 25°C | T1month 40°C | T9months 25°C | T9months 40°C |
|---|---|---|---|---|---|
| **Size (nm)** | 8 (96%) | 9 (90%) | 9 (90%) | 12 (83%) | 8 (94%) |
| | 2173 (3%) | 1773 (9%) | 1908 (9%) | 1984 (16%) | 1895 (5%) |
| **pH** | 5.98 | 6.20 | 5.92 | 5.76 | 5.72 |
| **Osmolality** | 293 | 291 | 292 | 285 | 289 |
| **Latanoprost assay % w/w (% from T0)** | 0.00504 | 0.00508 | 0.00521 | 0.00515 | 0.00517 |
| | | (100.8%) | (102.6%) | (101.4%) | (101.9%) |

### Example 4: Micellar Composition Comprising Cetalkonium Chloride (CKC) at 0.005% and Cremophor RH

A micellar composition (Micelle #4) was prepared that comprises 0.005% latanoprost, 0.1% Cremophor RH, 0.005% CKC, 0.9% NaCl, Tris Buffer pH 7.1, and water. Results of a stability analysis carried out for this composition are presented in Table 4. Micelle #4 is unpreserved according to European and US pharmacopeias.

**Table 4. Stability of Composition Micelle #4**

| **Parameters** | T0 | T1month 25°C | T1month 40°C | T9months 25°C | T9months 40°C |
|---|---|---|---|---|---|
| **Size (nm)** | 15 (95%) 1878 (4%) | 14 (100%) | 14 (95%) 2301 (4%) | 16 (95%) 1810 (4%) | 14 (100%) |
| **pH** | 7.04 | 6.93 | 6.84 | 6.75 | 6.75 |
| **Osmolality** | 318 | 306 | 306 | 301 | 305 |
| **Latanoprost assay % w/w (% from T0)** | 0.00504 | 0.00533 | 0.00567 | 0.00574 | 0.00547 |
| | | (106.2%) | (106.4%) | (107.6%) | (102.7%) |

### Example 5: Toxicity Study using an Acute Toxicity Model

***Animals and Eye Drop Treatments.*** All experiments were performed in accordance with the ARVO Statement for the Use of Animals in Ophthalmic and Vision Research. Albino 2- to 3-kg rabbits (New Zealand) of both sexes were used. Before all experimentations, the ocular surface integrity was examined by slip-lamp microscopy. A mixture of ketamine (35 mg/kg, Imalgène 500, Merial, Lyon, France) and xylazine (5 mg/kg, Bayer, Puteaux, France) was used to anesthetize the animals. Each group was composed of 7 rabbits: 5 rabbits were used for clinical and IVCM observation, conjunctival imprints collection at hour (H) 4, day (D) 1, D4 and D7; 2 rabbits in each treatment group were sacrificed for immunohistological procedures at D1, a time point chosen for the maximal inflammatory infiltration according to a preliminary seven days study (data not shown).

*50* µ*L* eye drops of sterile phosphate-buffered saline (PBS), 0.02%BAK solution (BAK Sol), 0.02%BAK in emulsion (BAK Em), 0.002%CKC solution (CKC Sol) or 0.002% CKC in emulsion (CKC Em) were instilled according to Ichijima H et al. (Cornea, 1992, 11: 221-225; erratum in: Cornea, 1992, 11: 368) in rabbit eyes 15 times at 5 minute-intervals.

All the eye drops were supplied by Novagali Pharma (Evry, France) and were sterile with physiological pH and osmolality. The present Applicants chose to compare 0.02% BAK to 0.002% CKC since these two QAC concentrations confer equivalent positive charge to the emulsion surface (zeta potential around 20 mV).

***Clinical findings and Draize Test*.** The first instillation was chosen as T0. During instillations, the time when conjunctival redness appeared was recorded. At H4, D1, D4, the eyes were examined using slit lamp microscopy for ocular irritation and scored according to a weighted scale for grading the severity of ocular lesions modified from Draize Test. In particular, the degree of redness, swelling (chemosis), and tearing for conjunctiva, the degree and area of cornea opacity, and increased prominence of folds and congestion of iris were evaluated. The possible maximum total score was 110 (conjunctiva=20, cornea=80, iris=10).

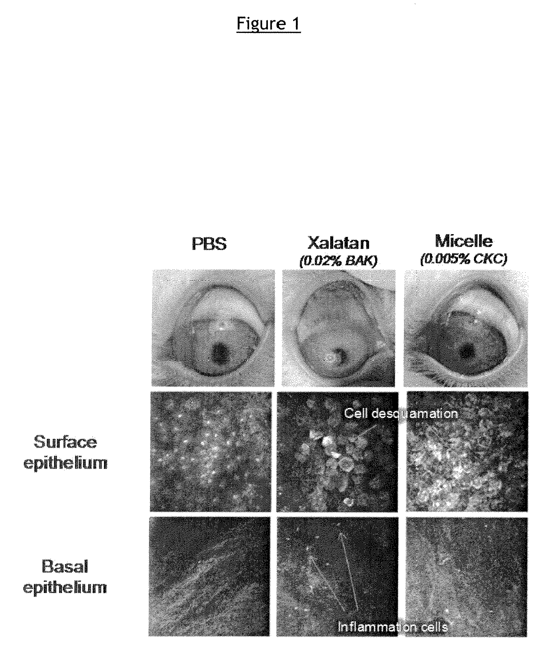

Figure 1 shows photographs of the treated eye of the three rabbits and microphotographs of epithelial cells in the cornea of these rabbits. Figure 1 clearly demonstrates that the inventive micellar composition is better tolerated than Xalatan. Rabbits administered the inventive micellar composition only exhibit a slight redness of the conjunctiva and very few inflammation cells.

***In vivo Confocal Microscopy Observation and Scoring.*** The laser scanning IVCM Heidelberg Retina Tomograph (HRT) II/Rostock Cornea Module (RCM, Heidelberg Engineering GmbH, Heidelberg, Germany), was used to examine the whole ocular surface (H. Liang et al., Mol. Vis., 2007, 13: 1169-1180; H. Liang et al., Mol. Vis., 2006, 12: 1392-1402; A. Labbe et al., J. Ocul. Pharmacol. Ther., 2006, 22: 267-278; A; Pauly et al., "New tools for the evaluation of toxic ocular surface changes in rat", Invest. Ophthalm. Vis. Sci., 2007, in press). The x-y position and the depth of the optical section were controlled manually; the focus position (µm) was automatically calculated by the HRT II/RCM. For all eyes, at least 10 confocal microscopic images of each layer in conjunctiva/limbus/cornea were recorded and analyzed. The final scores were the averages of the 10 eyes of 5 animals.

Scores were obtained for five zones: superficial epithelium, basal epithelium and anterior stroma of the cornea, limbus and conjunctival blood vessels. Cell morphology and nuclear aspects were evaluated, and the numbers of infiltrating inflammatory cells (lymphocytes, polymorphonuclear cells or dendritic-like cells) was assessed by using the Cell Count® program associated with the HRT II/RCM. The maximal score was 40.

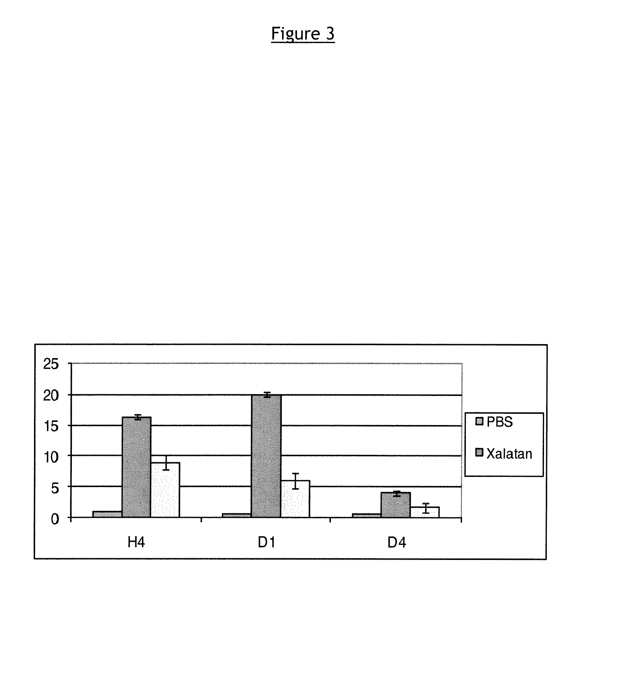

The HRT score obtained (see Figure 3) shows that only a few inflammation cells are present after instillation of the micelles.

***Flow cytometry analysis of rabbit impression cytology specimens*.** Conjunctival cells were extracted as previously described. Cells were extracted by gentle agitation for 30 minutes, centrifuged (1600 rpm, 5 minutes), and then analyzed on a flow cytometer (FC500, Beckman Coulter, Miami, FL, USA) equipped with an argon laser emitting at 488 nm, using the data analysis CXP software provided by the manufacturer. Direct immunofluorescence procedure was used to study the expressions of RLA DR (1:40, DakoCytomation, Clostrup, Denmark) and TNF-receptor 1 (mTNFR1, 1:40 dilution, R&D Systems, Minneapolis, MN). Mouse FITC-conjugated IgG1 (Lot: 28146, BD Biosciences Pharmingen, San Diego, CA) was used as negative control. For each antibody, a minimum of 1,000 conjunctival cells were analyzed and the results were expressed as percentages of positive cells.

Soon after the FCM analysis, the cell suspension was stained with propidium iodide (PI 0.5µg/mL, Sigma Chemical Co., USA). Immunoreactive cells were then spun down on a glass slide using a cytospin centrifuge (Shandon Cytospin 4, Thermo, Electron Corporation, Waltham, MA), and later observed and photographed under a confocal microscope (E800, PCM 2000, Nikon, Tokyo, Japan).

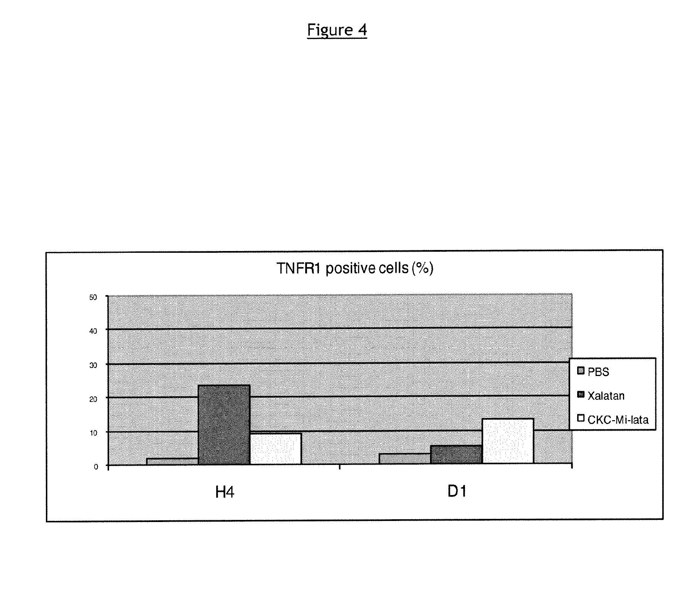

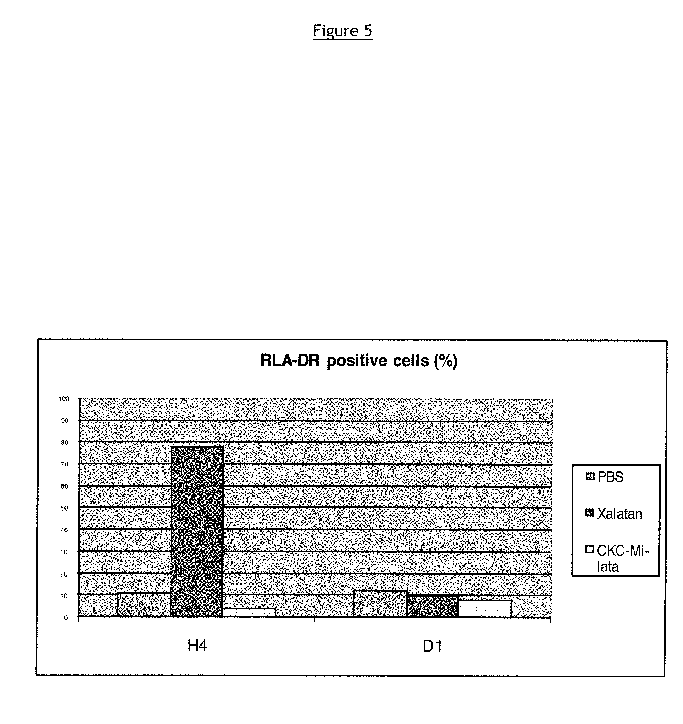

Figures 4 and 5 show that the inventive micellar composition induces less inflammation than Xalatan.

All the results obtained in the study of Example 5 demonstrate that the inventive micellar composition improves ocular tolerance of Latanoprost compared to the BAK micelles of Xalatan.

### Example 6: Micellar Composition Comprising Cetalkonium (CKC) at 0.005%, Cremophor RH40, and flurbiprofen, a non-steroidal anti-inflammatory drug

A micellar composition (Micelle #5) was prepared that comprises 0.03% fluriprofen, 0.1% Cremophor RH40, 0.005% CKC, and 2.5% glycerol in water. Results of a stability analysis carried out for this composition are presented in Table 5

**Table 5. Stability of Composition Micelle #5**

| **Parameters** | T0 | T1month 25°C | T1month 40°C | T3months 25°C | T3months 40°C |
|---|---|---|---|---|---|
| **Size (nm)** | 15 (33%) 239 (59%) 2180 (8%) | 16 (92%) 2067 (7%) | 16 (91%) 1906 (8%) | 14 (81%) 2488 (18%) | 24 (54%) 1531 (45%) |
| **pH** | 6.09 | 6.28 | 6.05 | 6.10 | 6.00 |
| **Osmolality** | 294 | 295 | 296 | 296 | 297 |

## Claims

1. Micellar composition for the treatment of an eye disease or condition, said composition comprising a mixture of n-alkyl dimethyl benzyl ammonium chlorides at a concentration equal or less than 0.02% in weight by weight of the total composition, and a pharmaceutically active substance, wherein the mixture of n-alkyl dimethyl benzyl ammonium chlorides comprises more than 30% n-alkyl dimethyl benzyl ammonium chlorides having a chain length superior or equal to C₁₆, and wherein the pharmaceutically active substance in the micellar composition is stabilized.

2. Micellar composition of claim **1,** wherein the mixture of n-alkyl dimethyl benzyl ammonium chlorides is present at a concentration equal or less than 0.01% in weight by weight of the total composition.

3. Micellar composition according to claim **1,** wherein the mixture of n-alkyl dimethyl benzyl ammonium chlorides is present at a concentration equal or less than 0.005% in weight by weight of the total composition.

4. Micellar composition according to anyone of claims **1** to **3,** wherein said composition comprises a plurality of populations of micelles and micelles of the main population have a size in the range of 1 nm to 100 nm.

5. Micellar composition according to anyone of claims **1** to **4** further comprising at least one pharmaceutically acceptable carrier or excipient.

6. Micellar composition according to claim **5, characterized in that** said composition has a positive zeta potential.

7. Micellar composition according to claim **6,** wherein the pharmaceutically acceptable carrier or excipient is a member of the group consisting of non-ionic surfactant, cationic surfactant, antioxidant, isotonicity agent, viscosifying agent, preservative, pH adjusting agent, buffering agent, osmotic agent, chelating agent, penetration enhancing agent, and any combination thereof.

8. Micellar composition according to claim **7,** wherein the non-ionic surfactant is tyloxapol, cremophor RH, cremophor E1, or a combination thereof.

9. Micellar composition according to claim **7,** wherein the osmotic agent is glycerol, sodium chloride, or a combination thereof.

10. Micellar composition according to anyone of claims **1** to **9,** wherein said composition is preserved.

11. Micellar composition according to anyone of claims **1** to **9,** wherein said composition is unpreserved.

12. Micellar composition according to anyone of claims **1** to **11,** wherein the pharmaceutically active substance is associated with micelles of n-alkyl dimethyl benzyl ammonium chlorides.

13. Micellar composition according to anyone of claims **1** to **11,** wherein the pharmaceutically active substance is substantially incorporated into micelles of n-alkyl dimethyl benzyl ammonium chlorides.

14. Micellar composition according to anyone of claims **1** to **13,** wherein the pharmaceutically active substance is selected from the group consisting of antibiotics, antiviral agents, antifungals, intraocular pressure lowering agents, non-steroidal anti-inflammatory agents, steroids, antiallergic agents, anti-angiogenic agents, biological agents, growth factors, immunomodulating agents, anti-glaucomateous agents, cytostatics, and any combination thereof.

15. Micellar composition according to anyone of claims **1** to **13,** wherein the pharmaceutically active substance is a prostaglandin.

16. Micellar composition according to claim **15,** wherein the prostaglandin is selected from the group consisting of latanoprost, unoprostone isopropyl, travoprost, bimatoprost, tafluprost, 8-isoprostaglandin E2, and any combination thereof.

17. Micellar composition according to claim **16,** wherein the prostaglandin is latanoprost.

18. Micellar composition according to claim **17,** wherein latanoprost is present at a concentration in the range of 0.001% to 0.01% in weight by weight of the total composition

19. Micellar composition according to claim **17,** wherein latanoprost is present at a concentration of 0.005% in weight by weight of the total composition.

20. Micellar composition according to anyone of claims 1 to 19, wherein the pharmaceutically active substance is stabilized for a period of time of more than about 1 year, preferably more than about 2 years, more preferably more than about 3 years, when stored at room temperature.

21. Medicament comprising an effective amount of a micellar composition according to anyone of claims **1** to **20.**

22. Use of a micellar composition according to anyone of claims **1** to **20** for the manufacture of a medicament for the treatment of an eye disease or condition.

23. Use according to claim **22,** wherein the eye disease or condition is a member of the group consisting of inflammation, allergy, dry eye, retinal diseases, infections, glaucoma, and ocular hypertension.
